# EUROPEAN PATENT APPLICATION

(11) **EP 4 477 177 A1**
(43) Date of publication of application: **18.12.2024**
(21) Application number: 24181673.5
(22) Date of filing: 12.06.2024
(51) Int. Cl.: A61B 34/30, A61M 25/09, A61M 25/01

(54) **SYSTEMS AND APPARATUSES FOR ELONGATED MEDICAL DEVICE TORQUERS**

(30) Priority: 14.06.2023 US 202318334593
(71) Applicant: Corindus Inc., Newton, MA 02466 (US)
(72) Inventor: ZIRPS, Christopher, Sharon, MA, 02067 (US)
(74) Representative: Horn Kleimann Waitzhofer Schmid-Dreyer Patent- und Rechtsanwälte PartG mbB

(57) **Abstract**

An apparatus for supporting an elongated medical device includes a first jaw having a first surface, a first pad arranged on the first surface of the first jaw, a second jaw having a second surface opposite the first surface, and a second pad arranged on the second surface of the second jaw. The first pad defines at least one first recess extending along at least a portion of a length of the first pad. The first pad and the second pad are configured to engage the elongated medical device. The at least one first recess is configured to maintain the elongated medical device at a center portion of the at least one first recess during movement of the elongated medical device.

## Description

### TECHNICAL FIELD

The present disclosure relates to systems and apparatuses for elongated medical device torquers.

### BACKGROUND

Catheters and other elongated medical devices (EMDs) may be used for minimally-invasive medical procedures for diagnosing and/or treating diseases of various vascular systems. Example medical procedures include neurovascular intervention (NVI) also known as neurointerventional surgery, percutaneous coronary intervention (PCI) and peripheral vascular intervention (PVI). These procedures typically involve navigating a guidewire through the vasculature to advance a catheter to deliver therapy. Robotic catheter-based procedure systems may be used to aid a physician in performing medical procedures such as those mentioned above.

### SUMMARY

At least one example embodiment relates to an apparatus for supporting an elongated medical device. The apparatus includes a first jaw having a first surface, a first pad arranged on the first surface of the first jaw, a second jaw having a second surface opposite the first surface, and a second pad arranged on the second surface of the second jaw. The first pad defines at least one first recess extending along at least a portion of a length of the first pad.

In at least one example embodiment, the first pad and the second pad are configured to engage the elongated medical device. The at least one first recess is configured to maintain the elongated medical device at a center portion of the at least one first recess during movement of the elongated medical device.

In at least one example embodiment, the movement of the elongated medical device includes one or more of axial movement, rotation, pinching, attachment, and clamping of the elongated medical device between the first jaw and the second jaw.

In at least one example embodiment, the at least one first recess has a concave or V-shape.

In at least one example embodiment, the second pad defines at least one second recess extending along at least a portion of a length of the second pad.

In at least one example embodiment, the at least one first recess includes a first curved surface extending in a longitudinal direction of the first pad and the at least one second recess includes a second curved surface extending in a longitudinal direcftion of the second pad.

In at least one example embodiment, a curvature of the first curved surface extends in a direction perpendicular to a longitudinal axis of the first pad and a curvature of the second curved surface extends in a direction perpendicular to a longitudinal axis of the second pad.

In at least one example embodiment, at least one of the first curved surface or the second curved surface has a constant radius of curvature.

In at least one example embodiment, at least a portion of at least one of the first pad or the second pad has a contoured surface, and the contoured surface has a cyclical pattern.

In at least one example embodiment, the cyclical pattern decreases from exterior edges of the first pad towards a center of the first pad and the cyclical pattern decreases from exterior edges of the second pad towards a center of the second pad.

In at least one example embodiment, the cyclical pattern of the first pad is 180° out of phase with the cyclical pattern of the second pad.

In at least one example embodiment, at least a portion of the first pad includes a first undulating surface extending along at least the portion of the length of the first pad and the at least one first recess is defined in the first undulating surface.

In at least one example embodiment, at least a portion of the second pad includes a second undulating surface extending along at least the portion of the length of the second pad and the at least one second recess is defined in the second undulating surface.

In at least one example embodiment, the first undulating surface includes a first plurality of waves and the second undulating surface includes a second plurality of waves. Peaks of the first plurality of waves are configured to align with valleys of the second plurality of waves and peaks of the second plurality of waves are configured to align with valleys of the first plurality of waves.

In at least one example embodiment, the apparatus includes a plurality of first recesses extending along at least the portion of the length of the first pad. Each of the plurality of first recesses include a curved or V-shaped groove.

In at least one example embodiment, the apparatus includes a plurality of second recesses extending along at least the portion of the length of the second pad. Each of the plurality of second recesses include a curved or V-shaped groove.

In at least one example embodiment, the plurality of first recesses align with the plurality of second recesses.

In at least one example embodiment, the plurality of first recesses are offset from the plurality of second recesses.

In at least one example embodiment, the apparatus includes a jaw assembly. In at least one example embodiment, the apparatus includes a first housing portion defining a first cavity, a jaw assembly configured to move within the first cavity, a second housing portion defining a second cavity, a pusher within the second cavity and configured to extend into at least a portion of the first housing portion, a biasing member engaged with the pusher and configured to apply a biasing force to the pusher, and an actuator engaged with the biasing member. The jaw assembly includes the first jaw, the first pad, the second jaw and the second pad. The second housing portion is configured to be coupled to a proximal end of the first housing portion. The pusher is configured to move the jaw assembly between a first position and a second position. The biasing member is engaged with the pusher and configured to apply a biasing force to the pusher. The actuator is configured to move the biasing member and the pusher in a first direction and a second direction.

At least one example embodiment relates to an apparatus for supporting an elongated medical device. The apparatus includes a first jaw having a first surface, a second jaw having a second surface, a first pad arranged on the first surface of the first jaw, and a second pad arranged on the second surface of the second jaw. The second surface faces the first surface of the first jaw. A first surface of the first pad faces a second surface of the second pad. The first pad includes a first wall extending at least from a first outer edge of the first surface of the first pad toward the second surface of the second pad and the second pad includes a second wall extending at least from a first outer edge of the second surface of the second pad toward the first surface of the first pad.

In at least one example embodiment, the first pad includes a third wall extending at least from a second outer edge of the first surface of the first pad toward the second surface of the second pad and the second pad includes a fourth wall extending at least from a second outer edge of the second surface of the second pad toward the first surface of the first pad.

In at least one example embodiment, the first wall extends along at least a portion of the first outer edge of the first surface of the first pad, the second wall extends along at least a portion of the first outer edge of the second surface of the second pad, the third wall extends along at least a portion of the second outer edge of the first surface of the first pad, and the fourth wall extends along at least a portion of the second outer edge of the second surface of the second pad.

In at least one example embodiment, the first wall, the second wall, the third wall and the fourth wall are castellated walls having a plurality of battlements.

In at least one example embodiment, the apparatus includes a first housing portion defining a first cavity, a jaw assembly configured to move within the first cavity, a second housing portion defining a second cavity, a pusher within the second cavity and configured to extend into at least a portion of the first housing portion, a biasing member engaged with the pusher and configured to apply a biasing force to the pusher, and an actuator engaged with the biasing member, the actuator configured to move the biasing member and the pusher in a first direction and a second direction. The jaw assembly includes the first jaw, the first pad, the second jaw and the second pad. The second housing portion is configured to be coupled to a proximal end of the first housing portion. The pusher is configured to move the jaw assembly between a first position and a second position.

In at least one example embodiment, the first pad and the second pad are configured to engage the elongated medical device and to maintain the elongated medical device at a center portion of the first pad and the second pad during movement of the elongated medical device.

At least one example embodiment relates to an apparatus for supporting an elongated medical device. The apparatus includes a first jaw having a first surface, a first pad arranged on the first surface of the first jaw, a second jaw having a second surface, and a second pad arranged on the second surface of the second jaw. The second surface is opposite the first surface. The first pad and the second pad are configured to maintain the elongated medical device at a center portion of the first pad and the second pad when engaged with the elongated medical device.

### BRIEF DESCRIPTION OF THE DRAWINGS

The various features and advantages of the non-limiting embodiments herein may become more apparent upon review of the detailed description in conjunction with the accompanying drawings. The accompanying drawings are merely provided for illustrative purposes and should not be interpreted to limit the scope of the claims. The accompanying drawings are not to be considered as drawn to scale unless explicitly noted. For purposes of clarity, various dimensions of the drawings may have been exaggerated.
FIG. 1 is a side, perspective view of a treatment system according to at least one example embodiment.
FIG. 2 is a block diagram of the treatment system of FIG. 1 according to at least one example embodiment.
FIG. 3A is an exploded view of a cassette assembly, robotic drive, and drive modules of the treatment system of FIG. 1 according to at least one example embodiment.
FIG. 3B is a side view of a torquer actuator and a cassette of FIG. 3A according to at least one example embodiment.
FIG. 3C is a cross-section of the torquer actuator and the cassette of FIG. 3B according to at least one example embodiment.
FIG. 4 is a perspective view of the torquer actuator of FIGS. 3B-3C according to at least one example embodiment.
FIG. 5 is an exploded view of the torquer actuator of FIG. 4 according to at least one example embodiment.
FIG 6. is cross-section view of the torquer actuator of FIG. 4 according to at least one example embodiment.
FIG. 7A is a perspective view of a jaw assembly of the torquer actuator of FIG. 5 according to at least one example embodiment.
FIG. 7B is a front view of the jaw assembly of FIG. 7A according to at least one example embodiment.
FIG. 7C front view of a jaw assembly according to at least one example embodiment.
FIG. 8A is a perspective view of a jaw assembly of FIG. 5 according to at least one example embodiment.
FIG. 8B is a perspective view of a second jaw of the jaw assembly of FIG. 8A according to at least one example embodiment.
FIG. 8C is a cross-section view of the jaw assembly of FIG. 8A according to at least one example embodiment.
FIG. 8D is a cross-section view of the jaw assembly of FIG. 8A according to at least one example embodiment.
FIG. 9A is a perspective view of the jaw assembly of FIG. 5 according to at least one example embodiment.
FIG. 9B is a front view of the jaw assembly of FIG. 9A according to at least one example embodiment.
FIG. 9C is a bottom, perspective view of a first jaw of the jaw assembly of FIG. 9A according to at least one example embodiment.
FIG. 10A is a perspective view of the jaw assembly of FIG. 5 according to at least one example embodiment.
FIG. 10B is a bottom, perspective view of a first jaw of the jaw assembly of FIG. 10 according to at least one example embodiment.
FIG. 10C is a front view of the jaw assembly of FIG. 10A according to at least one example embodiment.
FIG. 11A is a perspective view of the jaw assembly of FIG. 5 according to at least one example embodiment.
FIG. 11B is a bottom view of a first jaw of the jaw assembly of FIG. 11A according to at least one example embodiment.
FIG. 11C is a front view of the jaw assembly of FIG. 11A according to at least one example embodiment.
FIG. 12A is a perspective view of the jaw assembly of FIG. 5 according to at least one example embodiment.
FIG. 12B is a front view of the jaw assembly of FIG. 12A according to at least one example embodiment.

### DETAILED DESCRIPTION OF EXAMPLE EMBODIMENTS

Independent of the grammatical term usage, individuals with male, female or other gender identities are included within the term.

Some detailed example embodiments are disclosed herein. However, specific structural and functional details disclosed herein are merely representative for purposes of describing some example embodiments. Example embodiments may, however, be embodied in many alternate forms and should not be construed as limited to only example embodiments set forth herein.

Accordingly, while example embodiments are capable of various modifications and alternative forms, example embodiments thereof are shown by way of example in the drawings and will herein be described in detail. It should be understood, however, that there is no intent to limit an example embodiment to the particular forms disclosed, but to the contrary, example embodiments are to cover all modifications, combinations, equivalents, and alternatives falling within the scope of an example embodiment. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification. As used herein, the term "and/or" includes any and all combinations of one or more of the associated listed items.

It should be understood that, although the terms first, second, third, etc. may be used herein to describe various elements, regions, layers and/or sections, these elements, regions, layers, and/or sections should not be limited by these terms. These terms are only used to distinguish one element, region, layer, or section from another region, layer, or section. Thus, a first element, region, layer, or section discussed below could be termed a second element, region, layer, or section without departing from the teachings of example embodiment.

Spatially relative terms (e.g., "beneath," "below," "lower," "above," "upper," and the like) may be used herein for ease of description to describe one element or feature's relationship to another element(s) or feature(s) as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiment only and is not intended to be limiting of example embodiment. As used herein, the singular forms "a," "an," and "the" are intended to include the plural forms as well, unless the context clearly indicates otherwise. It will be further understood that the terms "includes," "including," "comprises," and/or "comprising," when used in this specification, specify the presence of stated features, integers, steps, operations, and/or elements, but do not preclude the presence or addition of one or more other features, integers, steps, operations, elements and/or groups thereof.

Example embodiments are described herein with reference to cross-sectional illustrations that are schematic illustrations of example embodiment. As such, variations from the shapes of the illustrations are to be expected. Thus, example embodiment should not be construed as limited to the shapes of regions illustrated herein but are to include deviations and variations in shapes.

When the words "about" and "substantially" are used in this specification in connection with a numerical value, it is intended that the associated numerical value include a tolerance of ±10% around the stated numerical value, unless otherwise explicitly defined. Moreover, when the terms "generally" or "substantially" are used in connection with geometric shapes, it is intended that precision of the geometric shape is not required but that latitude for the shape is within the scope of the disclosure. Furthermore, regardless of whether numerical values or shapes are modified as "about," "generally," or "substantially," it will be understood that these values and shapes should be construed as including a manufacturing or operational tolerance (e.g., ±10%) around the stated numerical values or shapes.

Unless otherwise defined, all terms (including technical and scientific terms) used herein have the same meaning as commonly understood by one of ordinary skill in the art to which example embodiment belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

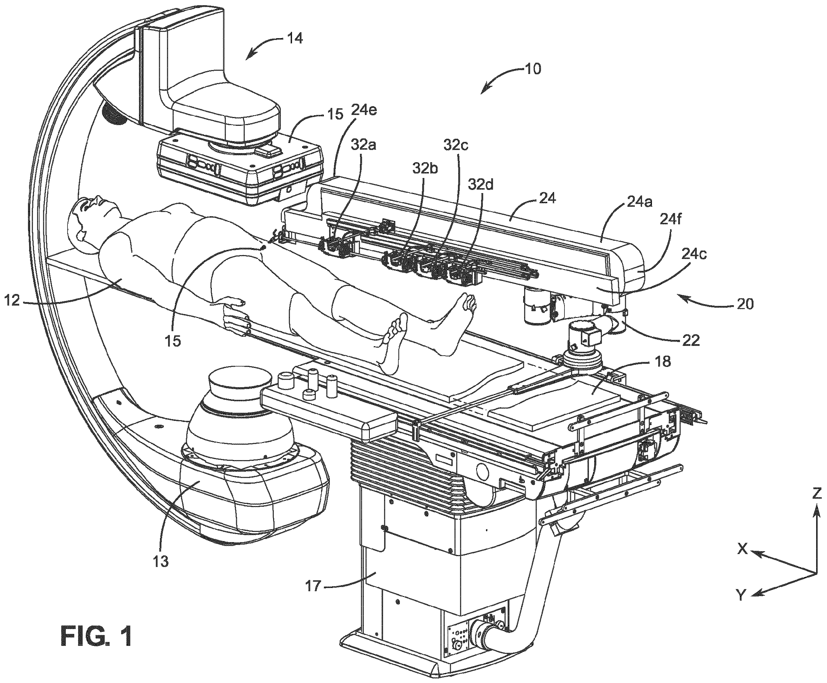

FIG. 1 is a side, perspective view of a treatment system according to at least one example embodiment.

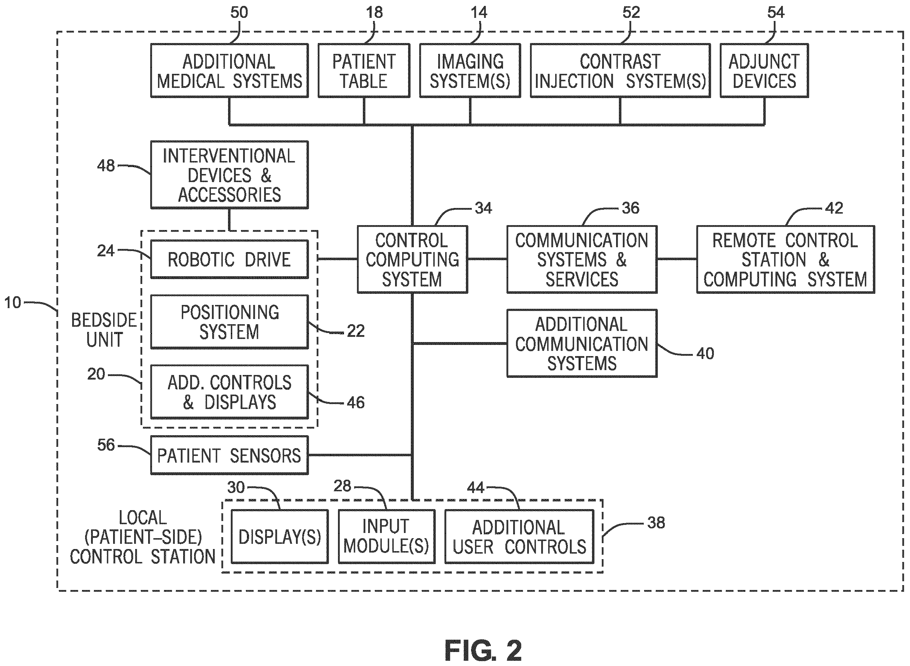

In at least one example embodiment, treatment system 10 may be used to perform catheter-based medical procedures, such as percutaneous intervention procedures including a percutaneous coronary intervention (PCI) (e.g., to treat STEMI), a neurovascular interventional procedure (NVI) (e.g., to treat an emergent large vessel occlusion (ELVO)), and/or peripheral vascular intervention procedures (PVI) (e.g., for critical limb ischemia (CLI), etc.). Catheter-based medical procedures may include diagnostic catheterization procedures during which one or more catheters or other elongated medical devices (EMDs) are used to aid in the diagnosis of a patient's disease. Catheter-based medical procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease. Therapeutic procedures may be enhanced by the inclusion of adjunct devices 54 (shown in FIG. 2) such as, intravascular ultrasound (IVUS), optical coherence tomography (OCT), fractional flow reserve (FFR), etc. In at least one example embodiment, percutaneous intervention devices or components (e.g., type of guidewire, type of catheter, etc.) may be selected based on the type of procedure that is to be performed.

In at least one example embodiment, a treatment system 10 includes a bedside unit 20 and at least one control station. The control station may include one or more of a local control station or a remote control station, such as a local control station 38 and/or a remote-control station 42 shown in FIG. 2. Bedside unit 20 includes a robotic drive 24 and a positioning system 22 adjacent a patient 12. The patient 12 may be supported on a patient table 18. A first end of the positioning system 22 may be attached to an end of the patient table 18, as shown in FIG. 1. In other example embodiments, the first end of the positioning system 22 may be attached to a base or a cart, for example. In at least one example embodiment, the positioning system 22 is used to position and support the robotic drive 24. A second end of the positioning system 22 may be attached to the robotic drive 24. The positioning system 22 may comprise a robotic arm, an articulated arm, a holder, etc. The positioning system 22 and/or the robotic drive 24 may be moved out of the way to allow for the patient 12 to be placed on the patient table 18. Once the patient 12 is positioned on the patient table 18, the positioning system 22 may be used to situate or position the robotic drive 24 relative to the patient 12 for a procedure or treatment. In at least one example embodiment, the patient table 18 may be supported by a pedestal 17, which may be secured to a floor of a room. The patient table 18 is configured to move with multiple degrees of freedom, for example, roll, pitch, and yaw, relative to the pedestal 17. In at least one example embodiment, the bedside unit 20 may include controls and displays 46, as shown in FIG. 2. For example, the controls and displays 46 may be located on a housing of the robotic drive 24 in some example embodiments.

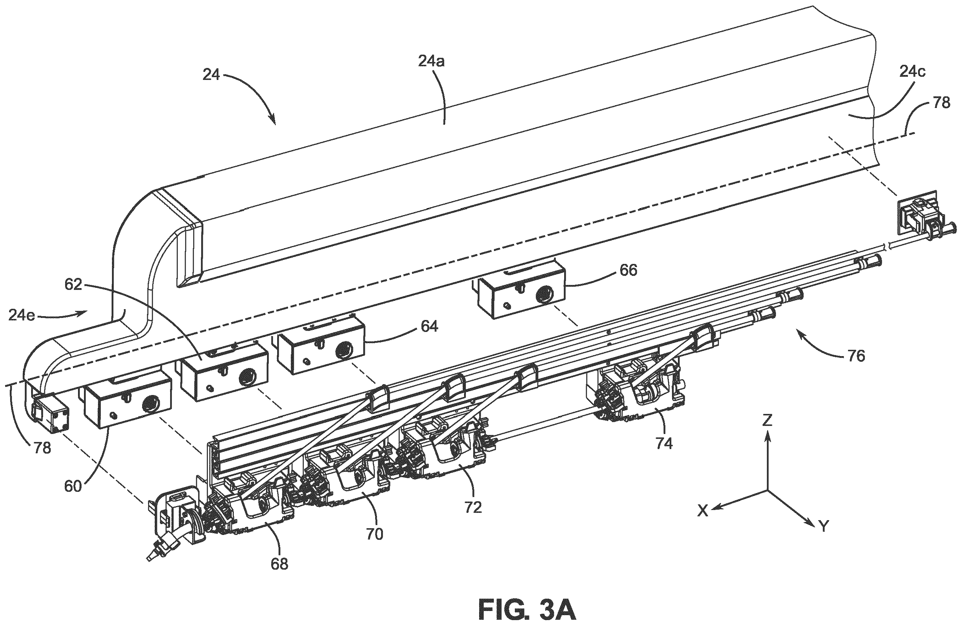

In at least one example embodiment, the robotic drive 24 may be equipped with one or more devices and accessories 48, as shown in FIG. 2. For example, the devices and accessories 48 may include one or more of guidewires, various types of catheters including but not limited to balloon catheters, stent delivery systems, stent retrievers, embolization coils, liquid embolics, aspiration pumps, device to deliver contrast media, medicine, hemostasis valve adapters, syringes, stopcocks, inflation device, etc. The one or more devices and accessories 48 may allow a user or operator to perform a procedure or treatment, such as a catheter-based medical procedure. In at least one example embodiment, the bedside unit 20 and/or the robotic drive 24 may include any number and/or combination of components to provide the bedside unit 20 with the functionality described herein. For example, the robotic drive 24 includes one or more device modules, such as a plurality of device modules 32a-d, mounted to a rail or linear member of the robotic drive 24. Each of the device modules 32a-d may be used to drive an elongated medical device ("EMD"), such as a catheter or guidewire. For example, the robotic drive 24 may be used to automatically feed a guidewire into a diagnostic catheter and into a guide catheter in an artery of the patient 12. One or more devices, such as an EMD, may enter the body (e.g., a vessel) of the patient 12 at an insertion point 16 via, for example, an introducer sheath. Each of the device modules 32a-d include a drive module and cassette removably attached to the drive module, as will be discussed below with respect to FIG. 3A. Each drive module is configured to move along a longitudinal axis of the robotic drive 24 with a bracket or stage.

In at least one example embodiment, the bedside unit 20 is in communication with the control station, allowing signals generated by the user inputs of the control station to be transmitted wirelessly or via hardwire to the bedside unit 20 to control various functions of bedside unit 20. For example, as shown in FIG. 2, the control computing system 34 may be coupled between the bedside unit 20 and the local control station 38 and/or the remote-control station 42. The bedside unit 20 may also provide feedback signals (e.g., loads, speeds, operating conditions, warning signals, error codes, etc.) to the control computing system 34. Communication between the control computing system 34 and various components of the treatment system 10 may be provided via a communication link that may be a wireless connection, cable connections, or any other means or mechanism capable of allowing communication to occur between components.

In at least one example embodiment, the control station may be located at a local site (e.g., the local control station 38 as shown in FIG. 2) or at a remote site (e.g., the remote-control station 42 shown in FIG. 2). For example, the treatment system 10 may be operated by the local control station 38, the remote-control station 42, or both the local control station 38 and the remote-control station 42. At the local site, a user or operator and the local control station 38 are located in the same room or an adjacent room to the patient 12 and bedside unit 20. For example, a local site is the location of the bedside unit 20 and a patient 12 or subject (e.g., animal or cadaver). At the remote site, the user or operator uses the remote-control station 42 to control the bedside unit 20 remotely. For example, the remote site does not have physical access to the bedside unit 20 and/or patient 12. The remote-control station 42 may be configured to communicate with the bedside unit 20 and/or the control computing system 34 at the local site using communication systems and services 36 (shown in FIG. 2), for example, through the Internet, a local area network (LAN), a wide area network (WAN), or other network.

In at least one example embodiment, the control station generally includes one or more input modules 28 configured to receive user inputs to operate various components or systems of treatment system 10. For example, the input modules 28 may be configured to cause the bedside unit 20 to perform various tasks using percutaneous intervention devices (e.g., EMDs) interfaced with the robotic drive 24 (e.g., to advance, retract, or rotate a guidewire, advance, retract or rotate a catheter, inflate or deflate a balloon located on a catheter, position and/or deploy a stent, position and/or deploy a stent retriever, position and/or deploy a coil, inject contrast media into a catheter, inject liquid embolics into a catheter, inject medicine or saline into a catheter, aspirate on a catheter, or to perform any other function that may be performed as part of a catheter-based medical procedure). The robotic drive 24 includes various drive mechanisms to cause movement (e.g., axial and/or rotational movement) of the components of the bedside unit 20 including the one or more devices and accessories 48.

In at least one example embodiment, the input modules 28 may include one or more touch screens, joysticks, scroll wheels, and/or buttons. In addition to input modules 28, the control station may use additional user controls 44, such as foot switches and microphones for voice commands, etc. The input modules 28 may be configured to advance, retract, or rotate various components and the one or more devices and accessories 48 such as, for example, a guidewire, and one or more catheters or microcatheters. Buttons may include, for example, an emergency stop button, a multiplier button, device selection buttons and automated move buttons. When an emergency stop button is pushed, the power (e.g., electrical power) is shut off or removed to bedside unit 20. When in a speed control mode, a multiplier button acts to increase or decrease the speed at which the associated component is moved in response to a manipulation of input modules 28. When in a position control mode, a multiplier button changes the mapping between input distance and the output commanded distance. Device selection buttons allow the user or operator to select which of the percutaneous intervention devices loaded into the robotic drive 24 are controlled by input modules 28. Automated move buttons are used to enable algorithmic movements that the treatment system 10 may perform on a percutaneous intervention device without direct command from the user or operator. In one embodiment, input modules 28 may include one or more controls or icons (not shown) displayed on a touch screen (that may or may not be part of a display), that, when activated, cause operation of a component of the treatment system 10. Input modules 28 may also include a balloon or stent control that is configured to inflate or deflate a balloon and/or deploy a stent. Each of the input modules 28 may include one or more buttons, scroll wheels, joysticks, touch screen, etc. that may be used to control the particular component or components to which the control is dedicated. In addition, one or more touch screens may display one or more icons (not shown) related to various portions of input modules 28 or to various components of treatment system 10.

In at least one example embodiment, the treatment system 10 includes an imaging system 14. For example, the imaging system 14 may include one or more of a non-digital X-ray, a digital X-ray, Computed Tomography (CT), Magnetic Resonance Imaging (MRI), ultrasound, etc. In at least one example embodiment, the imaging system 14 includes a digital X-ray imaging device in communication with the control station. In at least one example embodiment, the imaging system 14 may include a C-arm that allows imaging system 14 to partially or completely rotate around patient 12 in order to obtain images at different angular positions relative to patient 12 (e.g., sagittal views, caudal views, anterior-posterior views, etc.). In at least one example embodiment, the imaging system 14 may be a fluoroscopy system including a C-arm having an X-ray source 13 and a detector 15, also known as an image intensifier.

In at least one example embodiment, the imaging system 14 may be configured to take X-ray images of a desired area of the patient 12 during a procedure. For example, the imaging system 14 may be configured to take one or more X-ray images of the head to diagnose a neurovascular condition. The imaging system 14 may also be configured to take one or more X-ray images (e.g., real time images) during a catheter-based medical procedure to assist the user or operator of the control station to properly position a guidewire, guide catheter, microcatheter, stent retriever, coil, stent, balloon, etc. during the procedure. The image or images may be displayed on a display 30. For example, images may be displayed on the display 30 to allow the user or operator to accurately move a guide catheter or guidewire into a proper or desired position.

In at least one example embodiment, the robotic drive 24 includes a housing having a top or first member 24a; a bottom or second member opposite and parallel to the first member 24a; a front or third member 24c substantially perpendicular and extending between the first member 24a and the second member, and a fourth member opposite and parallel to the third member 24c and perpendicular to first member 24a and the second member. The third member 24c may be configured to face the user when the robotic drive 24 is in use. In at least one example embodiment, the robotic drive 24 includes a distal region 24e and a proximal region 24f opposite the distal region 24e.

FIG. 2 is a block diagram of the treatment system of FIG. 1 according to at least one example embodiment.

In at least one example embodiment, the control computing system 34 may be part of the control station, such as the local control station 38 and/or the remote-control station 42. The control computing system 34 may generally be an electronic control unit configured to provide the treatment system 10 with the various functionalities described herein. For example, the control computing system 34 may be an embedded system, a dedicated circuit, a general-purpose system programmed with the functionality described herein, etc. The control computing system 34 may be in communication with the bedside unit 20, the communications systems and services 36 (e.g., through Internet, firewalls, cloud services, session managers, a hospital network, etc.), the local control station 38, additional communications systems 40 (e.g., a telepresence system), the remote-control station 42, and patient sensors 56 (e.g., electrocardiogram (ECG) devices, electroencephalogram (EEG) devices, blood pressure monitors, temperature monitors, heart rate monitors, respiratory monitors, etc.). The control computing system 34 may also be in communication with the imaging system 14, the patient table 18, additional medical systems 50, contrast injection systems 52, and adjunct devices 54 (e.g., IVUS, OCT, FFR, etc.).

In at least one example embodiment, the control computing system 34 is configured to generate control signals based on the user's interaction with input modules 28 (e.g., of a control station such as a local control station 38 or a remote-control station 42) and/or based on information accessible to control computing system 34 such that a medical procedure may be performed using the treatment system 10. For example, the control computing system 34 may be in communication with the bedside unit 20, the robotic drive 24, the positioning system 22, and the additional controls and displays 46 and may provide control signals to one or more of the bedside unit 20, the robotic drive 24, the positioning system 22, and additional controls and displays 46 to control the operation of the motors and drive mechanisms used to drive the percutaneous intervention devices (e.g., guidewire, catheter, etc.). For example, the various drive mechanisms may be part of the robotic drive 24. The local control station 38 may include one or more displays 30, one or more input modules 28, and the additional user controls 44.

The remote-control station 42 and the control computing system 34 may include similar or analogous components as the local control station 38. The remote-control station 42 and the local control station 38 may be different and tailored based on their required functionalities. In at least one example embodiment, the additional user controls 44 include one or more foot input controls. The foot input controls may be configured to allow the user to select functions of the imaging system 14, such as turning on and off the X-ray and scrolling through different stored images. In another example embodiment, the foot input controls may be configured to allow the user to select which devices are mapped to scroll wheels included in input modules 28. In at least one example embodiment, additional communication systems 40, such as audio and/or video communications, may be employed to help the operator interact with the patient, medical staff, and/or equipment in the vicinity of the bedside.

FIG. 3A is an exploded view of a cassette assembly, robotic drive, and drive modules of the treatment system of FIG. 1 according to at least one example embodiment.

Referring to FIG. 3A, in at least one example embodiment, the device module 32a includes a first drive module 60 and a first cassette 68, the device module 32b includes a second drive module 62 and a second cassette 70, the device module 32c includes a third drive module 64 and a third cassette 72, and the device module 32d includes a fourth drive module 66 and a fourth cassette 74. The first cassette 68, the second cassette 70, the third cassette 72, and the fourth cassette 74 may form a multi-unit cassette assembly 76. Each of the first cassette 68, the second cassette 70, the third cassette 72, and the fourth cassette 74 may be configured to be removably coupled to the first drive module 60, the second drive module 62, the third drive module 64, and the fourth drive module 66, respectively.

In at least one example embodiment, each of the plurality of device modules 32a-d are configured to move independently of each other along a longitudinal axis 78 extending through a length of the robotic drive 24 from the distal region 24e to the proximal region 24f.

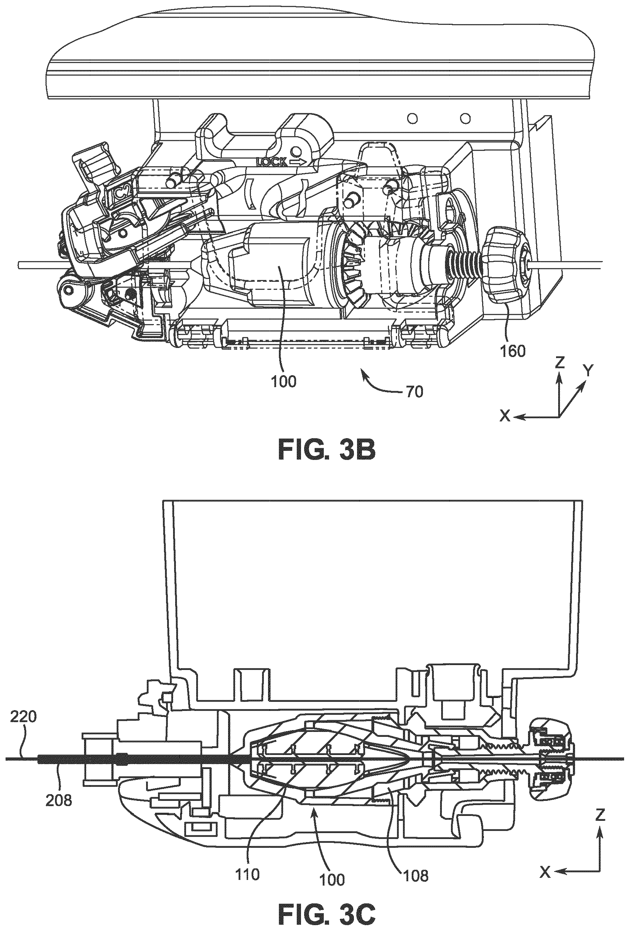

FIG. 3B is a side view of a torquer actuator and a cassette of FIG. 3A according to at least one example embodiment. FIG. 3C is a cross-section of the torquer actuator and the cassette of FIG. 3B according to at least one example embodiment.

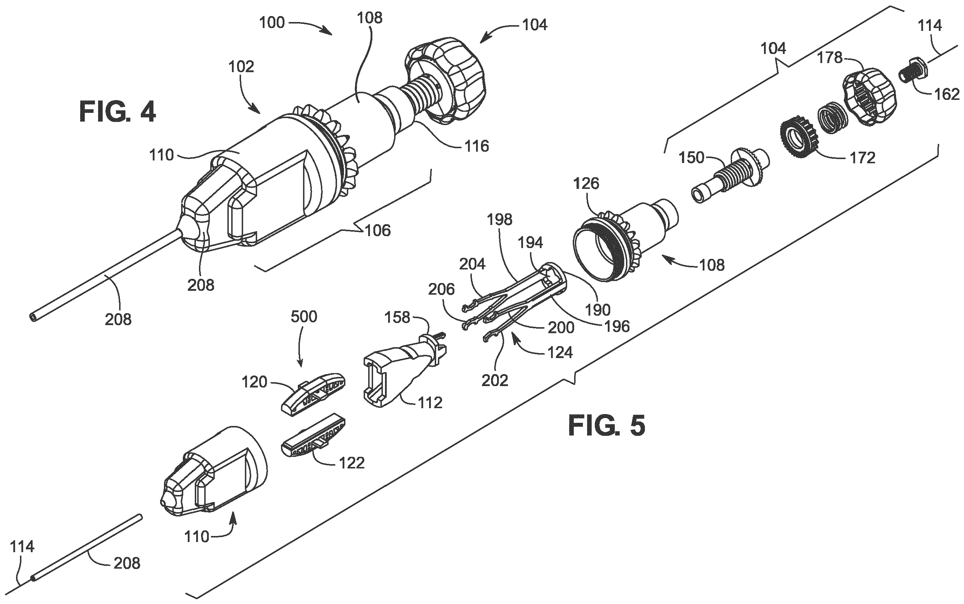

Referring to FIGS. 3B and 3C, in at least one example embodiment, a torquer actuator 100 is configured to be positioned in one or more of the first cassette 68, the second cassette 70, the third cassette 72, and the fourth cassette 74. For example purposes, FIG. 3B illustrates the torquer actuator 100 positioned in the second cassette 70. The torquer actuator 100 may include a guide tube 208 configured to receive an EMD 220. The torquer actuator 100 may also be configured to secure the EMD 220 within a first housing portion 110 and a second housing portion 108 of the torquer actuator 100, as will be described below with respect to FIGS. 4-6.

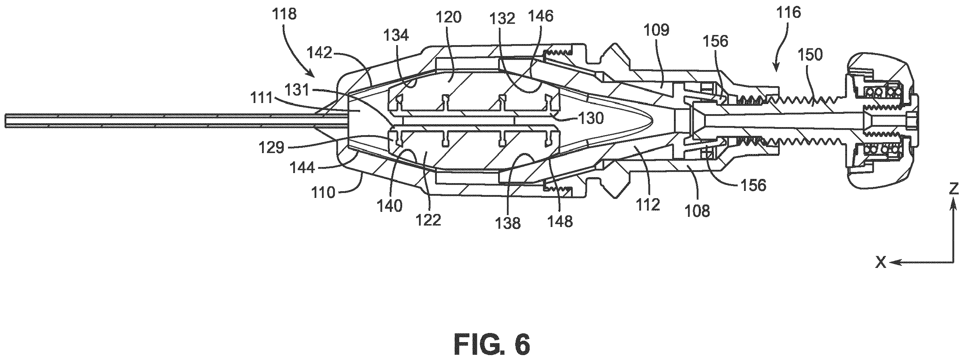

FIG. 4 is a perspective view of the torquer actuator of FIGS. 3B-3C according to at least one example embodiment. FIG. 5 is an exploded view of the torquer actuator of FIG. 4 according to at least one example embodiment. FIG 6. is cross-section view of the torquer actuator of FIG. 4 according to at least one example embodiment.

Referring to FIGS. 4-6, in at least one example embodiment, the torquer actuator includes a housing 106 including the first housing portion 110 and the second housing portion 108. A pusher 112 may be movably received within the housing 106 along a torquer longitudinal axis 114 between a proximal end 116 and a distal end 118 of housing 106. In at least one example embodiment, a jaw assembly 500 is positioned within the housing 106 and configured to releasably pinch a shaft of the EMD 220. For example, the jaw assembly 500 includes a first jaw 120 and a second jaw 122. The first jaw 120 and the second jaw 122 may be configured to move between a first position and a second position, such as towards and away from each other, to releasably pinch and un-pinch the EMD 220. The jaw assembly 500 is an example of the above mentioned apparatus for supporting an elongated medical device.

In at least one example embodiment, a biasing member 124 is configured to move towards and away from the jaw assembly 500 to move the first jaw 120 and the second jaw 122 between the first position and the second position. For example, movement of the pusher 112 from the proximal end 116 towards the distal end 118 may move the first jaw 120 and the second jaw 122 towards each other, such as towards the torquer longitudinal axis 114, and movement of the pusher 112 from the distal end 118 towards the proximal end 116 may move the first jaw 120 and the second jaw 122 away from each other, such as away from the torquer longitudinal axis 114. In other example embodiments, movement of the pusher 112 from the proximal end 116 towards the distal end 118 may move the first jaw 120 towards the second jaw 122 (e.g., while the second jaw 122 is stationary relative to the first jaw 120) or may move the second jaw 122 towards the first jaw 120 (e.g., while the first jaw 120 is stationary relative to the second jaw 122).

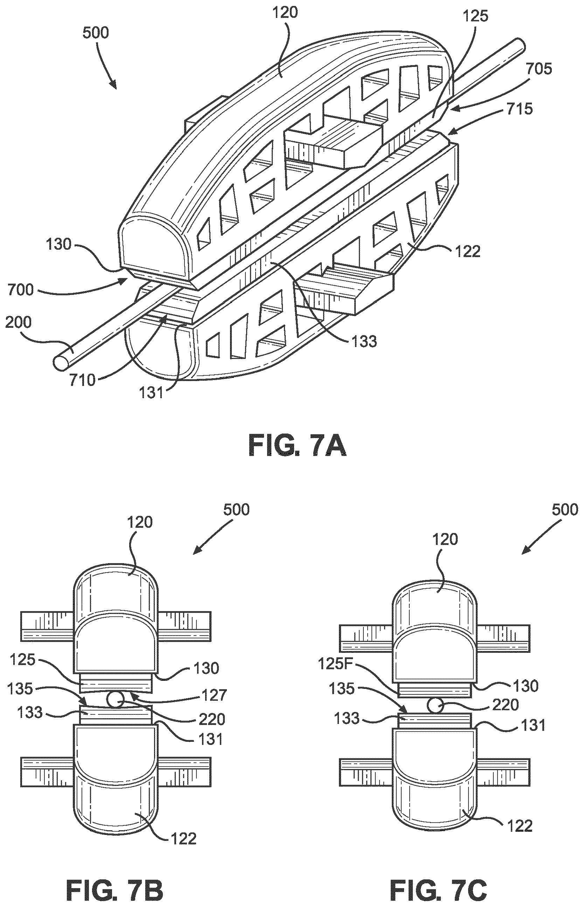

In at least one example embodiment, the first jaw 120 includes a first surface 130 and the second jaw 122 includes a second surface 131. The first surface 130 is configured to face the second surface 131. In at least one example embodiment, the first surface 130 has a first pad arranged thereon and the second surface 131 has a second pad arranged thereon. Surfaces of the first pad and the second pad are configured to contact or engage the EMD 220. For clarity, the first pad and the second pad are not shown in FIGS. 4-6. However, example embodiments of the first pad and the second pad will be discussed in more detail later with regard to FIGS. 7A-12B.

In at least one example embodiment, the first housing portion 110 includes a first ramp 142 and a second ramp 144. The pusher 112 includes a first ramp 146 and a second ramp 148. As the pusher 112 moves from the proximal end 116 towards the distal end 118, the first ramp 146 of the pusher 112 contacts a first proximal ramp 132 of the first jaw 120 and the second ramp 148 of the pusher 112 contacts a second proximal ramp 138 of the second jaw 122. Similarly, a first distal ramp 134 of the first jaw 120 contacts the first ramp 142 of the first housing portion and a second distal ramp 140 of the second jaw 122 contacts the second ramp 144 of the first housing portion 110. Such contact moves the first jaw 120 and the second jaw 122 towards each other in a generally perpendicular direction towards the torquer longitudinal axis 114, which is configured to pinch the EMD 220 between the first pad and the second pad.

In at least one example embodiment, the pusher 112 is configured to move within the first housing portion 110 by manipulation of an actuator 104. The actuator 104 includes a shaft 150 configured to be threadedly engaged with the second housing portion 108. A distal end of the shaft 150 is configured to be coupled to at least a proximal end 158 of the pusher 112 such that movement of the shaft 150 in a distal direction moves the pusher 112 in the distal direction, such as towards the distal end 118 of the housing 106. Additionally, movement of the shaft 150 in a proximal direction moves the pusher 112 in the proximal direction, such as towards the proximal end 116 of the housing 106. In at least one example embodiment, the pusher 112 includes a pair of arms 156 configured to engage the distal end of the shaft 150 such that when the shaft 150 is moved in the proximal direction, the pusher 112 also moves in the proximal direction.

In at least one example embodiment, the actuator 104 includes a knob 160 that is secured to a proximal end of the shaft 150 opposite the pusher 112. The knob 160 may be secured to the shaft 150 with a fastener 162. Rotation of the knob 160 in a first direction results in rotation of a drive gear 172. The drive gear 172 may be engaged with the proximal end of the shaft 150 such that rotation of the knob 160 and the drive gear 172 rotates the shaft 150 in the first direction. In at least one example embodiment, a biasing member 178 is positioned between the knob 160 and the drive gear 172. The biasing member 178 is configured to bias the drive gear 172 into engagement with the shaft 150.

Still referring to FIGS. 4-6, in at least one example embodiment, as the knob 160 is rotated in the first direction, the shaft 150 moves in the distal direction, such as towards the distal end 118 of the housing 106. Such movement in the distal direction also moves the pusher 112 in the distal direction towards the distal end 118 of the housing which causes the first jaw 120 and the second jaw 122 to move toward one another to pinch the EMD 220. In at least one example embodiment, each of the knob 160, the drive gear 172, and the shaft 150 are configured to rotate about the torquer longitudinal axis 114.

In at least one example embodiment, rotation in the first direction may be in the clockwise direction. The knob 160, the drive gear 172, and the shaft 150 may also be configured to rotate in a second direction, such as a counter-clockwise direction. As the knob 160 is rotated in the second direction, or the counter-clockwise direction, the shaft 150 may move in the proximal direction. Such movement in the proximal direction also moves the pusher 112 in the proximal direction towards the proximal end 116 of the housing 106, which causes the first jaw 120 and the second jaw 122 to move away from each other. As the first jaw 120 and the second jaw 122 move away from each other, the EMD 220 may be inserted into the torquer actuator 100 or the EMD 220 may be released and removed from the torquer actuator 100.

In at least one example embodiment, the biasing member 124 includes a base portion 190 having an aperture 194. The aperture 194 is configured to receive at least a portion of the shaft 150. The shaft 150 and the biasing member 124 are free to move along torquer longitudinal axis 114 independently of one another. The biasing member 124 includes a first arm 196 and a second arm 198 that are spaced from one another and spaced apart from the torquer longitudinal axis 114. The first arm 196 and the second arm 198 extend along the exterior of the pusher 112. The first arm 196 includes a first branch 200 and a second branch 202 configured to engage a portion of the first jaw 120 and a portion of the second jaw 122, respectively, on a first side. Similarly, the second arm 198 includes a first branch 204 and a second branch 206 configured to engage a portion of the first jaw 120 and the second jaw 122, respectively, on a second side opposite the first side. The branches 200, 202, 204 and 206 are configured to bias the first jaw 120 and the second jaw 122 towards and away from each other. For example, the shaft 150 is configured to move the biasing member 124, including the branches 200, 202, 204, and 206, in the distal direction towards the distal end 118 of the housing 106 to move the first jaw 120 and the second jaw 122 towards each other and to move the biasing member 124 in the proximal direction towards the proximal end 116 of the housing 106 to move the first jaw 120 and the second jaw 122 away from each other.

Operation of a torquer for an elongated medical device, such as the torquer actuator 100, is also described in International Patent Application Publication No. WO 2022/154977, entitled "TORQUER FOR AN ELONGATED MEDICAL DEVICE," filed on January 14, 2021, the entire contents of which are herein incorporated by reference.

As discussed in more detail below, one or more example embodiments provide pad configurations for a jaw assembly of a torquer actuator that mitigate (e.g., substantial) lateral motion of EMDs, encourage centering of the EMD along the longitudinal axis of the jaw assembly, and/or reduce the likelihood of contact of the EMD with surfaces other than the pads of the jaw assembly.

One or more example embodiments may also enable more economical manufacture of a torque device, which has both relatively high torque/force performance, while also mitigating damage to the EMDs being manipulated.

FIG. 7A is a perspective view of a jaw assembly of the torquer actuator of FIG. 5 according to at least one example embodiment. FIG. 7B is a front view of the jaw assembly of FIG. 7A according to at least one example embodiment.

Referring to FIGS. 7A and 7B, in at least one example embodiment, the jaw assembly 500 includes a first pad 125 arranged on the first surface 130 of the first jaw 120 and a second pad 133 arranged on the second surface 131 of the second jaw 122. The second surface 131 of the second jaw 122 is opposite the first surface 130 of the first jaw 120. The first pad 125 may define at least one first channel or recess 127 extending along at least a portion of a length of the first pad 125. For example, the first pad 125 may define a recess 127 that may extend from a first end 700 to a second end 705 of the first pad 125. The second pad 133 may define at least one second channel or recess 135 extending along at least a portion of a length of the second pad 133. For example, the at least one second recess 135 may extend from a first end 710 to a second end 715 of the second pad 133.

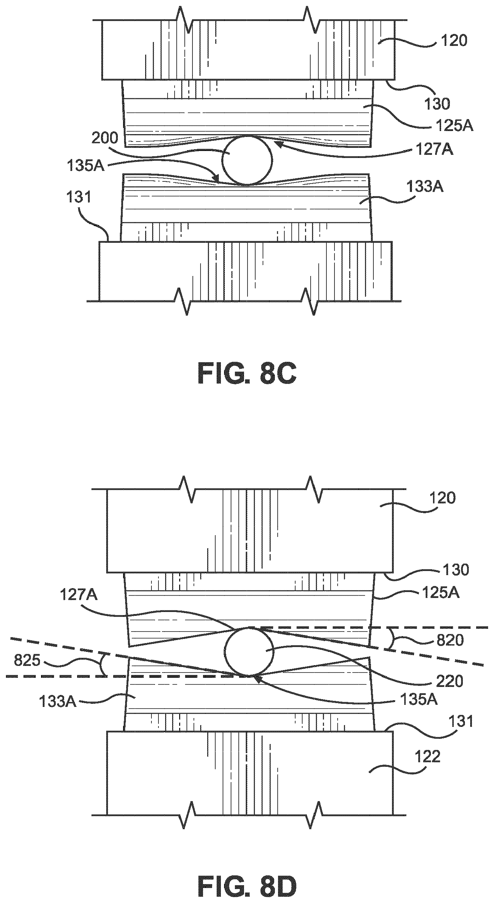

The first recess 127 may include a first curved (concave) surface extending in a longitudinal direction of the first pad 125 and the second recess 135 may include a second curved (concave) surface extending in a longitudinal direction of the second pad 133. The curvature of the first curved surface may extend in a direction perpendicular to the longitudinal axis of the first pad 125 and the curvature of the second curved surface may extend in a direction perpendicular to the longitudinal axis of the second pad 133. In at least one example embodiment, the first curved surface and/or the second curved surface may have a constant radius of curvature, which may be the same or different as that discussed later with regard to the example embodiment shown in FIG. 8C. In another example embodiment the first curved surface and/or the second curved surface may be linear, as discussed later with regard to the example embodiment shown in FIG. 8D. For example, the first curved surface of the first pad 125 and the second curved surface of the second pad 133 may form a first angle and a second angle, respectively. In this case, one or more of the recesses may have a V-shape, rather than be curved.

In an example, the first recess 127 and/or the second recess 135 may extend continuously along the entire length of the first pad 125 and/or the second pad 133, respectively. Alternatively, the first recess 127 and/or the second recess 135 may be discontinuous in that there may be breaks along the length of the first pad 125 and/or the second pad 133. In yet another example, the first recess 127 and/or the second recess 135 may not extend to the ends of the respective pads.

In at least one example embodiment, the first end 700 and/or the second end 705 of the first pad 125 may have a beveled or tapered edge that slopes downward towards the first surface 130. Additionally, the first end 710 and/or the second end 715 of the second pad 133 may have a beveled or tapered edge that slopes downward towards the second surface 131, as shown in FIG. 7A.

In at least one example embodiment, the first pad 125 and the second pad 133 are configured to engage the EMD 220. In at least one example embodiment, the at least one first recess 127 and the at least one second recess 135 are configured to maintain the EMD 220 at center portions of the first pad 125 and the second pad 133 during movement of the EMD 220. For example, the at least one first recess 127 and the at least one second recess 135 are configured to pinch, attach, engage, and/or clamp the EMD 220 between the center portions of the first pad 125 and the second pad 133 of the first jaw 120 and the second jaw 122. Movement of the EMD 220 may include one or more of pinching, attachment, and clamping of the EMD 220 between the first jaw 120 and the second jaw 122. Movement of the EMD 220 may also include axial or linear movement along a longitudinal axis of the EMD 220 and rotation of the EMD 220 while performing a procedure. In at least some example embodiments discussed herein, the center or center portions of the first pad 125 and the second pad 133 may refer to center portions along a longitudinal axis of the jaw assembly.

In at least one example embodiment, an outer surface of the EMD 220 may include a coating. For example, the EMD 220 may include a lubricious, hydrophilic coating over an elastomer coated super-elastic core. Such a coating may be slippery when wet which, along with the super-elastic core, may cause the EMD 220 to slide off axis during movement of the EMD 220. In other example embodiments, the EMD 220 may comprise a polytetrafluoroethylene (PTFE) coating on a metal core. In still other example embodiment, the EMD 220 may comprise a metallic or bare metal material. As discussed above, the at least one first recess 127 and the at least one second recess 135 may be configured to maintain the EMD 220 at center portions of the first pad 125 and the second pad 133 during movement of the EMD 220 regardless of the coating applied or the material composition of the EMD 220.

FIG. 7C is front view of another jaw assembly according to at least one example embodiment. The jaw assembly shown in FIG. 7C is similar to the jaw assembly shown in FIG. 7B, except that only one of the first and second pads includes a recess while the other pad has a flat or substantially flat surface (without a recess). Because of the similarities between these embodiments, only differences will be discussed herein.

Referring to FIG. 7C, the first pad 125F may include a flat or substantially flat surface, rather than the first recess 127 shown in FIG. 7B. The EMD 220 may be positioned between the first pad 125F and the at least one second recess 135 of the second pad 133, as shown in FIG. 7C. Although not shown, in other example embodiments, the second pad 133 may have a flat or substantially flat surface and the first pad 125F may have a recess that is the same as or similar to the recess 127 shown in FIG. 7B. In this case, the second pad 133 may be the same as or similar to the first pad 125F shown in FIG. 7C. In such embodiments, the second pad 133 may not include the at least one second recess 135 and the EMD 220 may be positioned between the first recess 127 and the flat surface of the second pad 133.

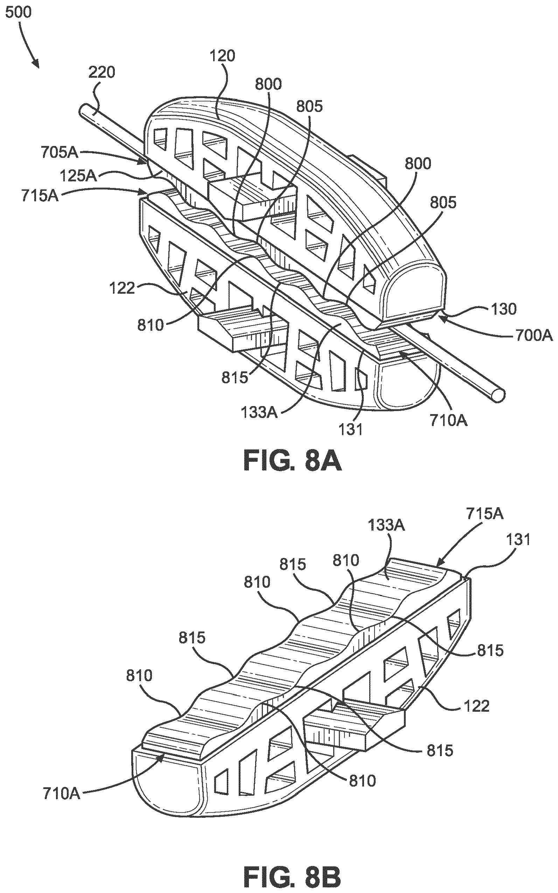

FIG. 8A is a perspective view of a jaw assembly of FIG. 5 according to at least one example embodiment. FIG. 8B is a perspective view of a second jaw of the jaw assembly of FIG. 8A according to at least one example embodiment. FIG. 8C is a cross-section view of the jaw assembly of FIG. 8A according to at least one example embodiment. FIG. 8D is a cross-section view of the jaw assembly of FIG. 8A according to at least one example embodiment. According to at least this example embodiment, the first jaw may be the same as the second jaw, but oriented in a different manner.

Referring to FIGS. 8A-8D, in at least one example embodiment, the jaw assembly 500 includes a first pad 125A and a second pad 133A. In at least one example embodiment, the first pad 125A and the second pad 133A may each include a contoured surface. For example, the contoured surface may include a cyclical pattern. In at least one example embodiment, the first pad 125A includes a first undulating surface extending along at least a portion of the length of the first pad 125A and the second pad 133A includes a second undulating surface extending at least a portion of the length of the second pad 133A. As shown in at least FIGS. 8A-8B, for example, the first pad 125A includes a first undulating surface extending from a first end 700A to a second end 705A of the first pad 125A and the second pad 133A includes a second undulating surface extending from a first end 710A to a second end 715A of the second pad 133A. In one example, the first undulating surface and/or the second undulating surface may extend continuously along the entire length of the first pad 125A and/or the second pad 133A, respectively. Alternatively, the first undulating surface and/or the second undulating surface may be discontinuous in that there may be breaks along the length of the first pad 125A and/or the second pad 133A. In at least one example embodiment, the cyclical pattern or the first undulating surface of the first pad 125A is configured to be out of phase with the cyclical pattern or the second undulating surface of the second pad 133A. For example, the first undulating surface of the first pad 125A may be about 180° out of phase with the second undulating surface of the second pad 133A. In other example embodiments, the first undulating surface of the first pad 125A may be less than about 180° out of phase with the second undulating surface of the second pad 133A. However, example embodiments should not be limited to these examples.

According to at least some example embodiments, the cyclical pattern or the first undulating surface of the first pad 125A may gradually decrease from exterior edges of the first pad 125A towards a center of the first pad 125A to define a first recess 127A. The cyclical pattern or the second undulating surface of the second pad 133A may also gradually decrease from exterior edges of the second pad 133A towards a center of the second pad 133A to define a second recess 135A. In at least one example embodiment, the cyclical pattern or the first undulating surface of the first pad 125A and/or the cyclical pattern or the second undulating surface of the second pad 133A may vary along the length of the first pad 125A and the second pad 133A. For example, one or more of an amplitude or a wavelength of the first undulating surface of the first pad 125A may vary along the length of the first pad 125A and/or one or more of an amplitude or a wavelength of the second undulating surface of the second pad 133A may vary along the length of the second pad 133A.

In at least one example embodiment, the first recess 127A includes a first curved surface extending in a longitudinal direction of the first pad 125A and the second recess 135A includes a second curved surface extending in a longitudinal direction of the second pad 133A. The curvature of the first curved surface may extend in a direction perpendicular to the longitudinal axis of the first pad 125A and the curvature of the second curved surface may extend in a direction perpendicular to the longitudinal axis of the second pad 133A. In an example embodiment the first curved surface and/or the second curved surface may be linear, as shown in FIG. 8D. For example, the first curved surface of the first pad 125A and the second curved surface of the second pad 133A may form a first angle 820 and a second angle 825, respectively. The first angle 820 and the second angle 825 may be between about 6 degrees and about 9 degrees in some example embodiments. In at least one example embodiment, the first curved surface and/or the second curved surface may have a constant radius of curvature. In this case, the angle of contact with the EMD may be constant moving away from the center of the respective surface.

In another example embodiment, as shown in FIG. 8C, the first curved surface and/or the second curved surface may have a varying radius of curvature, wherein the angle of contact with the EMD 220 may increase or decrease moving away from the center of the respective surface.

In at least one example embodiment, the first undulating surface of the first pad 125A includes a first plurality of waves having peaks 800 and valleys 805. The second undulating surface of the second pad 133A includes a second plurality of waves having peaks 810 and valleys 815. In at least one example embodiment the peaks 800 of the first plurality of waves of the first pad 125A are configured to align with the valleys 815 of the second plurality of waves of the second pad 133A. The peaks 810 of the second plurality of waves of the second pad 133A may also be configured to aligned with the valleys 805 of the first plurality of waves of the first pad 125A.

In another example embodiment, the first pad 125A including the first undulating surface and the second pad 133A including the second undulating surface may be the same. In this example, when the first pad 125A is placed above or on top of the second pad 133A, the valleys 805 of the first plurality of waves of the first pad 125A may align with the valleys 815 of the second plurality of waves of the second pad 133A. In at least one example embodiment, each of the first pad 125A and the second pad 133A may include about five peaks and about five valleys. In other example embodiments, each of the first pad 125A and the second pad 133A may include greater than or less than five peaks and five valleys.

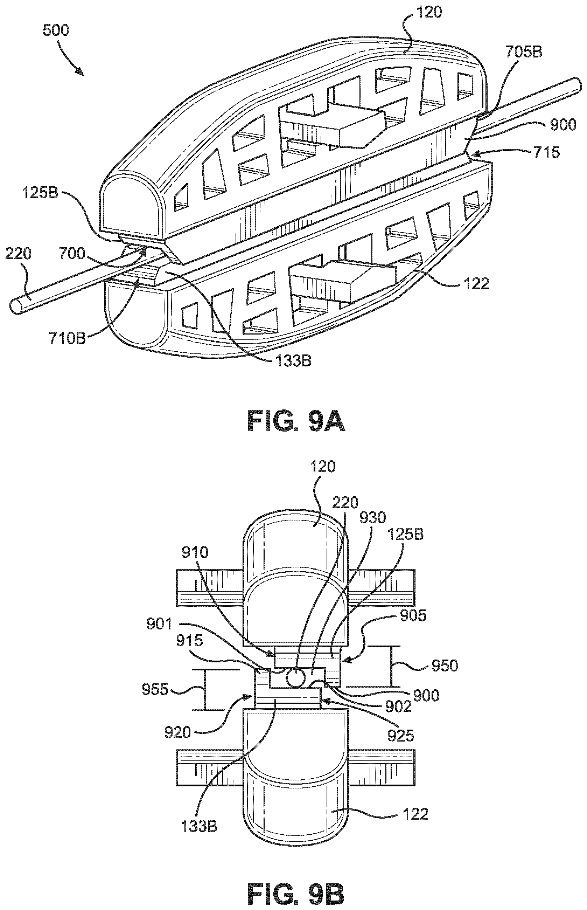

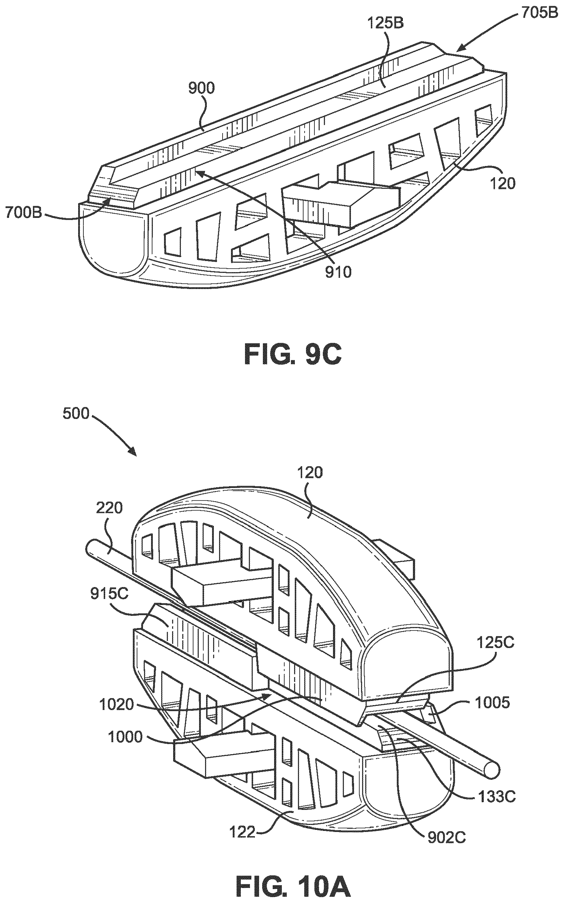

FIG. 9A is a perspective view of the jaw assembly of FIG. 5 according to at least one example embodiment. FIG. 9B is a front view of the jaw assembly of FIG. 9A according to at least one example embodiment. FIG. 9C is a bottom, perspective view of a first jaw of the jaw assembly of FIG. 9A according to at least one example embodiment.

Referring to FIGS. 9A-9C, in at least one example embodiment, the jaw assembly 500 includes a first pad 125B and a second pad 133B. In at least one example embodiment, a first surface 901 of the first pad 125B faces a second surface 902 of the second pad 133B. The first pad 125B may include a first wall 900 extending from at least a portion of a first outer edge 905 of the first pad 125B towards the second surface 902 of the second pad 133B. The second pad 133B may include a second wall 915 extending from at least a portion of a first outer edge 920 of the second pad 133B towards the first surface 901 of the first pad 125B. As shown in FIGS. 9A-9C, for example, the first wall 900 may extend from the first outer edge 905 towards the second surface 902 along substantially the entire length of the first pad 125B. Similarly, the second wall 915 may extend from the first outer edge 920 towards the first surface 901 along substantially the entire length of the first pad 125B. In at least one example embodiment, the first wall 900 and the second wall 915 may be formed from the same material as the first pad 125B and the second pad 133B. For example, the first wall 900 and the first pad 125 may be unitary and the second wall and the second pad 133B may be unitary. In other example embodiments, the first wall 900 and the second wall 915 may be formed from the same material as the first jaw 120 and the second jaw 122.

In at least one example embodiment, the first wall 900 and the second wall 915 define a channel 930. For example, the channel 930 may be defined between the first surface 901 and the first wall 900 of the first pad 125B and the second surface 902 and the second wall 915 of the second pad 133B. The channel 930 may be configured to receive at least a portion of the EMD 220 and maintain the EMD 220 at a center portion between the first pad 125B and the second pad 133B. For example, the channel 930 may be configured to maintain the EMD 230 at a center between the first pad 125B and the second pad 133, and between the first wall 900 and the second wall 915, during movement of the EMD 220. Movement of the EMD 220 may include one or more of pinching, attachment, and clamping of the EMD 220 between the first jaw 120 and the second jaw 122. Movement of the EMD 220 may also include axial or linear movement along a longitudinal axis of the EMD 220 and rotation of the EMD 220 while performing a procedure.

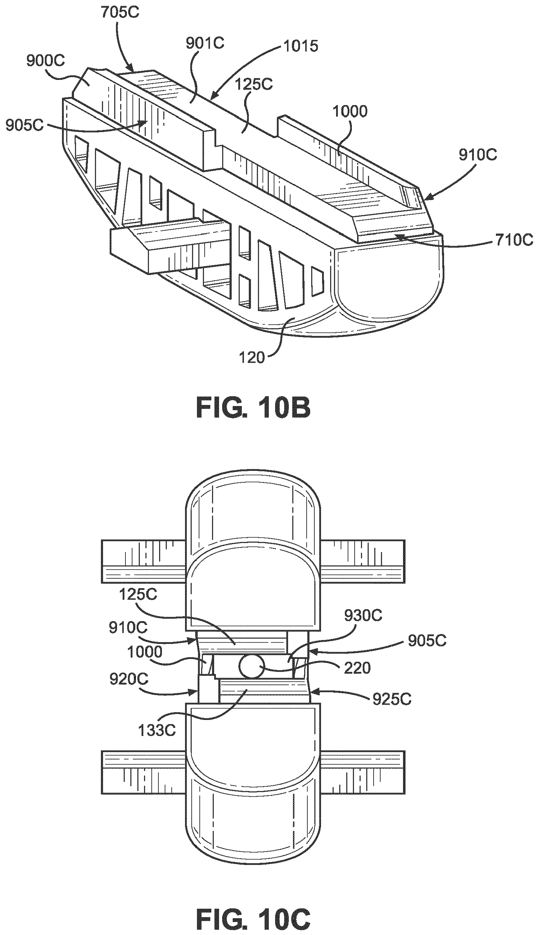

In at least one example embodiment, the first wall 900 may extend along at least a portion of a length of the first pad 125B in the longitudinal direction. For example, the first wall 900 may extend the length of the first pad 125B from a first end 700B to a second end 705B opposite the first end 700B, as shown in FIG. 9C. In other example embodiments, the first wall 900 may extend along (e.g., only) a portion of the length of the first pad 125B from the first end 700B to the second end 705B or from the second end 705B to the first end 700B, as will be discussed below with respect to FIG. 10B. Similarly, the second wall 915 of the second pad 133B may extend the length of the second pad 133B in the longitudinal direction from a first end 710B to a second end 715B opposite the first end 710B. In other example embodiments, the second wall 915 of the second pad 133B may extend along (e.g., only) a portion of the length of the second pad 133B from the first end 710B to the second end 715B or from the second end 715B to the first end 710B.

In at least one example embodiment, an interior surface of the first wall 900 may align with the second outer edge of 925 of the second pad 133B, as shown in FIG. 9B. Similarly, an interior surface of the second wall 915 may align with the second outer edge 910 of the first pad 125B. In other example embodiments, the first wall 900 may extend towards the second surface 902 of the second pad 133B. For example, an end portion of the first wall 900 may face and/or be in contact with at least a portion of the second surface 902 of the second pad 133B. Similarly, the second wall 915 may extend to the first surface 901 of the first pad 125B. For example, an end portion of the second wall 915 may face and/or be in contact with the first surface 901 of the first pad 125B.

In at least one example embodiment, the first wall 900 has a first height 950 and the second wall 915 has a second height 955. The first height 950 and the second height 955 may be the same, substantially the same, or equal in some example embodiments. For example, the first height 950 and the second height 955 may be between about 0.75 mm and about 1.5 mm. In other example embodiments, the first height 950 and the second height 955 may be different. For example, the first height 950 may be greater than the second height 955 or the second height 955 may be greater than the first height 950.

FIG. 10A is a perspective view of the jaw assembly of FIG. 5 according to at least one example embodiment. FIG. 10B is a bottom, perspective view of a first jaw of the jaw assembly of FIG. 10A according to at least one example embodiment. FIG. 10C is a front view of the jaw assembly of FIG. 10A according to at least one example embodiment.

Referring to FIGS. 10A-10C, in at least one example embodiment, the jaw assembly 500 includes a first pad 125C and a second pad 133C. In at least one example embodiment, the first pad 125C may include a first wall 900C extending from a portion of a first surface 901C and a third wall 1000 extending from a portion of the first surface 901C. The third wall 1000 may be opposite the first wall 900C. For example, the third wall 1000 may be adjacent a second outer edge 910C of the first pad 125C and the first wall 900C may be adjacent a first outer edge 905C of the first pad 125C, as shown in FIG. 10B. Additionally, the first wall 900C and the third wall 1000 may be positioned on opposite ends of the first pad 125C. For example, the first wall 900C may be adjacent a second end 705C of the first pad 125C and the third wall 1000 may be adjacent a first end 700C of the first pad 125C, as shown in FIG. 10B. In other example embodiments, the first wall 900C may be adjacent the first end 700C and the third wall 1000 may be adjacent the second end 705C of the first pad 125C.

In at least one example embodiment, the second pad 133C includes a second wall 915C extending from a portion of a second surface 902C of the second pad 133C and a fourth wall 1005 extending from a portion of the second surface 902C. In at least one example embodiment, the second wall 915C and the fourth wall 1005 of the second pad 133C may be similar or analogous to the first wall 900C and the third wall 1000 of the first pad 125C. The fourth wall 1005 may be opposite the second wall 915C. For example, the fourth wall 1005 may be adjacent a second outer edge 925C of the second pad 133C and the second wall 915C may be adjacent the first outer edge 920C of the second pad 133C. Additionally, the second wall 915C and the fourth wall 1005 may be positioned on opposite ends of the second pad 133C. For example, the second wall 915C may be adjacent a second end 715C of the second pad 133C and the fourth wall 1005 may be adjacent a first end 710C of the second pad 133C, as shown in FIG. 10A. In other example embodiments, the second wall 915C may be adjacent the first end 710C and the fourth wall 1005 may be adjacent the second end 715C of the second pad 133C.

Although the first wall 900C, the second wall 915C, the third wall 1000 and the fourth wall 1005 are illustrated as extending about half the length of the respective pads in FIGS. 10A-10C, example embodiments should not be limited to this example. Rather, in another example, each of the walls 900C, 915C, 1000 and 1005 may extend more or less than half, so long as the aggregate length of the walls extending along a respective side of the first pad 125C and the second pad 133C is about the length of the first pad 125C or the second pad 133C. Additionally, the respective walls need not extend to the end of a respective pad.

In at least one example embodiment, a portion of the first pad 125C and the second pad 133C may be removed or omitted to receive at least a portion of at least one of the walls of the second pad 133C and the first pad 125C, respectively. For example, a portion of the first pad 125C adjacent the first wall 900C may be removed or omitted to form a first recess 1010 and a portion of the first pad 125C adjacent the third wall 1000 may be removed or omitted to form a second recess 1015, as shown in FIG. 10B. The first recess 1010 may be adjacent the first end 700C of the first pad 125C opposite the first wall 900C and the second recess 1015 may be adjacent the second end 705C of the first pad 125C opposite the third wall 1000. In at least one example embodiment, the first recess 1010 may be configured to receive the second wall 915C of the second pad 133C and the second recess 1015 may be configured to receive the fourth wall 1005 of the second pad 133C. In at least one example embodiment, the second pad 133C may be similar or analogous to the first pad 125C. For example, the second pad 133C may define a third recess 1020 adjacent the first end 710C of the second pad 133C opposite the second wall 915C and configured to receive the third wall 1000 of the first pad 125C, as shown in FIG. 10A. The second pad 133C may also define a fourth recess (not shown) adjacent the second end 715C of the second pad 133C opposite the fourth wall 1005 and configured to receive the first wall 900C of the first pad 125C.

In at least one example embodiment, the first wall 900C and the third wall 1000 of the first pad 125C and the second wall 915C and the fourth wall 1005 of the second pad 133C define a channel 930C. For example, the channel 930C may be defined between the first surface 901C, the first wall 900C, the third wall 1000, the second surface 902C, the second wall 915C, and the fourth wall 1005. The channel 930C may be configured to receive at least a portion of the EMD 220 and maintain the EMD 220 at a center portion between the first pad 125C and the second pad 133C. For example, the channel 930C may be configured to maintain the EMD 230 at a center between the first pad 125C and the second pad 133C, and between the first wall 900C and the second wall 915C, during movement of the EMD 220. Movement of the EMD 220 may include one or more of pinching, attachment, and clamping of the EMD 220 between the first jaw 120 and the second jaw 122. Movement of the EMD 220 may also include axial or linear movement along a longitudinal axis of the EMD 220 and rotation of the EMD 220 while performing a procedure.

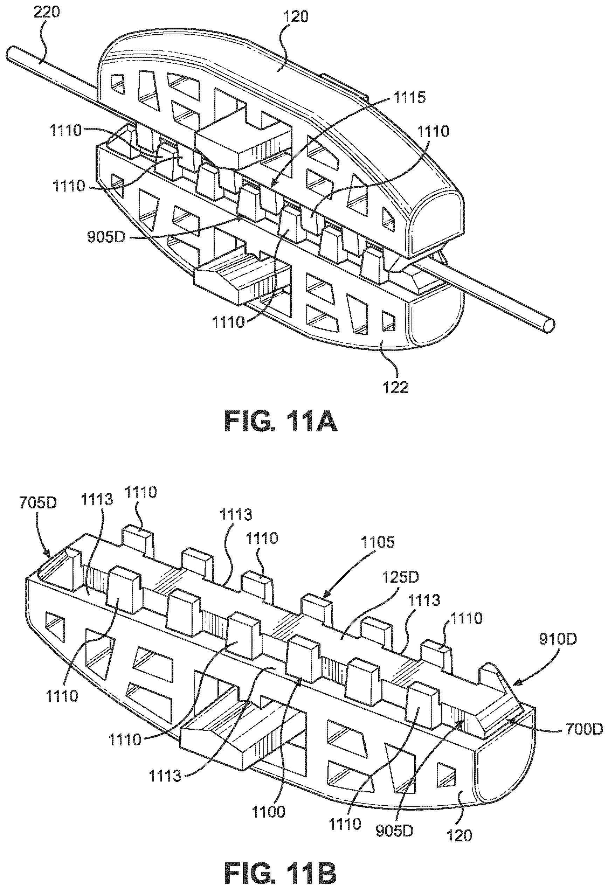

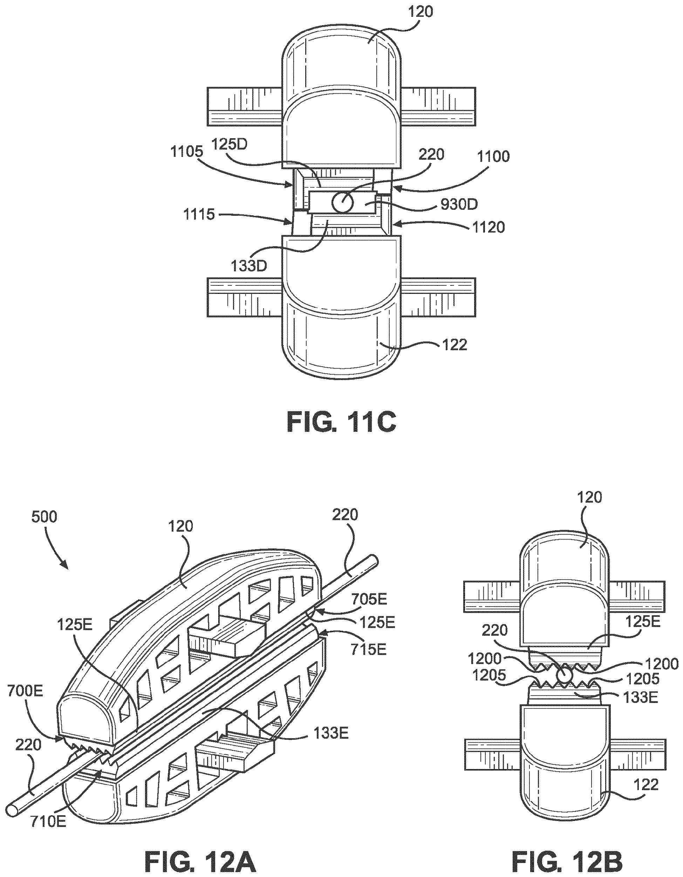

FIG. 11A is a perspective view of the jaw assembly of FIG. 5 according to at least one example embodiment. FIG. 11B is a bottom view of a first jaw of the jaw assembly of FIG. 11A according to at least one example embodiment. FIG. 11C is a front view of the jaw assembly of FIG. 11A according to at least one example embodiment.

Referring to FIGS. 11A-11C, in at least one example embodiment, the jaw assembly 500 includes a first pad 125D and a second pad 133D. In at least one example embodiment, the first pad 125D includes a first wall 1100 adjacent a first outer edge 905D and a second wall 1105 adjacent a second outer edge 910D. The first wall 1100 may extend along at least a portion of the first outer edge 905D and the second wall 1105 may extend along at least a portion of the second outer edge 910D. As shown in FIGS. 11A-11C, for example, the first wall 1100 and the second wall 1105 may extend from a first end 700D to a second end 705D of the first pad 125D (e.g., substantially the entire length of the first pad 125D). Example embodiments should not, however, be limited to this example. For example, the first wall 1100 and the second wall 1105 need not extend to ends of the first pad 125D.

In at least one example embodiment, the first wall 1100 and the second wall 1105 are castellated walls, as shown in FIG. 11B. For example, the castellated walls of the first wall 1100 and the second wall 1105 include a plurality of battlements 1110 and a plurality of recesses 1113 between each of the plurality of battlements 1110.

In at least one example embodiment, the second pad 133D includes a third wall 1115 and a fourth wall 1120. The third wall 1115 and the fourth wall 1120 of the second pad 133D may be similar or analogous to the first wall 1100 and the second wall 1105 of the first pad 125D discussed above with respect to FIG. 11B. For example, the third wall 1115 and the fourth wall 1120 may be castellated walls having the plurality of battlements 1110 and the plurality of recesses 1113 between each of the plurality of battlements 1110.

In at least one example embodiment, the castellated walls of the first wall 1100 and the second wall 1105 may be offset from the castellated walls of the third wall 1115 and the fourth wall 1120, as shown in FIG. 11A. For example, the plurality of battlements 1110 of the first wall 1100 and the second wall 1105 may be configured to be received by the plurality of recess 1113 of the third wall 1115 and the fourth wall 1120. Additionally, the plurality of battlements 1110 of the third wall 1115 and the fourth wall 1120 may be received by the plurality of recesses 1113 of the first wall 1100 and the second wall 1105.

In at least one example embodiment, the first pad 125D, including the first wall 1100 and the second wall 1105, and the second pad 133D, including the third wall 1115 and the fourth wall 1120, define a channel 930D. The channel 930D may be configured to receive at least a portion of the EMD 220 and maintain the EMD 220 at a center portion between the first pad 125D and the second pad 133D. For example, the channel 930D may be configured to maintain the EMD 230 at a center between the first pad 125D and the second pad 133D, and between the walls 1100, 1105, 1115, and 1120, during movement of the EMD 220. Movement of the EMD 220 may include one or more of pinching, attachment, and clamping of the EMD 220 between the first jaw 120 and the second jaw 122. Movement of the EMD 220 may also include axial or linear movement along a longitudinal axis of the EMD 220 and rotation of the EMD 220 while performing a procedure.

FIG. 12A is a perspective view of the jaw assembly of FIG. 5 according to at least one example embodiment. FIG. 12B is a front view of the jaw assembly of FIG. 12A according to at least one example embodiment.

In at least one example embodiment, the jaw assembly 500 includes a first pad 125E and a second pad 133E. In at least one example embodiment, the first pad 125E defines a plurality of first recesses 1200 extending along at least a portion of the length of the first pad 125E and the second pad 133E defines a plurality of second recesses 1205 extending along at least a portion of the length of the second pad 133E. For example, as shown in FIGS. 12A-12B, the plurality of first recesses 1200 may extend from a first end 700E to a second end 705E of the first pad 125E and the plurality of second recesses 1205 may extend from a first end 710E to a second end 715E of the second pad 133E. Each of the plurality of first recesses 1200 and each of the plurality of second recesses 1205 may comprise a V-shaped groove, as shown in FIG. 12B. In other example embodiments, each of the plurality of first recesses 1200 and each of the plurality of second recesses 1205 may comprise a curved (or concave) groove having a U-shape.

In at least one example embodiment, the plurality of first recesses 1200 may be aligned with the plurality of second recesses 1205, as shown in FIG. 12B. In other example embodiments, the plurality of first recesses 1200 may be offset from the plurality of second recesses 1205. At least a portion of the plurality of first recesses 1200 and the plurality of second recesses 1205 may be configured to maintain the EMD 220 at a center portion of the first pad 125E and the second pad 133E during movement of the elongated medical device. Movement of the EMD 220 may include one or more of pinching, attachment, and clamping of the EMD 220 between the first jaw 120 and the second jaw 122. Movement of the EMD 220 may also include axial or linear movement along a longitudinal axis of the EMD 220 and rotation of the EMD 220 while performing a procedure.

According to example embodiments, the plurality of first recesses 1200 and the plurality of second recesses 1205 may extend continuously along the entire length of the first pad 125E and/or the second pad 133E, respectively. Alternatively, the plurality of first recesses 1200 and the plurality of second recesses 1205 may be discontinuous in that there may be breaks along the length of the first pad 125E and/or the second pad 133E. In yet another example, the plurality of first recesses 1200 and the plurality of second recesses 1205 may not extend to the ends of the respective pads.

Although example embodiments may be described herein with regard to each of the pads of the jaw assembly 500 including a particular pad configuration, example embodiments should not be limited to this example. Rather, one pad of the jaw assembly 500 may include a particular pad configuration as described with regard to FIGS. 7A-12B and another pad of the jaw assembly 500 may have a different pad configuration from among those described with regard to FIGS. 7A- 12B. In another example embodiment, only one of the pads of the jaw assembly 500 may have a pad configuration as described with regard to FIGS. 7A-12B and the other pad may, for example, have a flat or substantially flat surface.

Example embodiments have been disclosed herein, it should be understood that other variations may be possible. Such variations are not to be regarded as a departure from the spirit and scope of the present disclosure, and all such modifications as would be obvious to one skilled in the art are intended to be included within the scope of the following claims.

## Claims

1. An apparatus (500) for supporting an elongated medical device (220), the apparatus (500) **characterized by**:
a first jaw (120) having a first surface (130);
a first pad (125) arranged on the first surface (130) of the first jaw (120), the first pad (125) defining at least one first recess (127) extending along at least a portion of a length of the first pad (125);
a second jaw (122) having a second surface (131), the second surface (131) being opposite the first surface (130); and
a second pad (133) arranged on the second surface (131) of the second jaw (122).

2. The apparatus (500) of claim 1, **characterized in that**:
the first pad (125) and the second pad (133) are configured to engage the elongated medical device (220);
the at least one first recess (127) is configured to maintain the elongated medical device (220) at a center portion of the at least one first recess (127) during movement of the elongated medical device (220); and
the movement of the elongated medical device (220) includes one or more of axial movement, rotation, pinching, attachment, and clamping of the elongated medical device (220) between the first jaw (120) and the second jaw (122).

3. The apparatus (500) of any of claims 1 or 2, **characterized in that** the at least one first recess (127) has a concave or V-shape.

4. The apparatus (500) of any of the preceding claims, **characterized in that**:
the second pad (133) defines at least one second recess (135) extending along at least a portion of a length of the second pad (133);
the at least one first recess (127) includes a first curved surface extending in a longitudinal direction of the first pad (125);
the at least one second recess (135) includes a second curved surface extending in a longitudinal direction of the second pad (133);
a curvature of the first curved surface extends in a direction perpendicular to a longitudinal axis of the first pad (125);
a curvature of the second curved surface extends in a direction perpendicular to a longitudinal axis of the second pad (133); and
at least one of the first curved surface or the second curved surface has a constant radius of curvature.

5. The apparatus (500) of any of the preceding claims, **characterized in that**:
at least a portion of at least one of the first pad (125) or the second pad (133) has a contoured surface; and
the contoured surface has a cyclical pattern.

6. The apparatus (500) of claim 5, **characterized in that**:
the cyclical pattern decreases from exterior edges of the first pad (125) towards a center of the first pad (125);
the cyclical pattern decreases from exterior edges of the second pad (133) towards a center of the second pad (133); and
the cyclical pattern of the first pad (125) is 180° out of phase with the cyclical pattern of the second pad (133).

7. The apparatus (500) of claim 5, **characterized in that**:
at least a portion of the first pad (125) includes a first undulating surface extending along at least the portion of the length of the first pad (125);
the at least one first recess (127) is defined in the first undulating surface;
at least a portion of the second pad (133) includes a second undulating surface extending along at least the portion of the length of the second pad (133); and
the at least one second recess (135) is defined in the second undulating surface.

8. The apparatus (500) of claim 7, **characterized in that**:
the first undulating surface includes a first plurality of waves;
the second undulating surface includes a second plurality of waves;
peaks (800) of the first plurality of waves are configured to align with valleys (815) of the second plurality of waves; and
peaks (810) of the second plurality of waves are configured to align with valleys (805) of the first plurality of waves.

9. The apparatus (500) of claim 1, further **characterized by**:
a plurality of first recesses (1200) extending along at least the portion of the length of the first pad (125), each of the plurality of first recesses (1200) being a curved or V-shaped groove; and
a plurality of second recesses (1205) extending along at least the portion of the length of the second pad (133), each of the plurality of second recesses (1205) being a curved or V-shaped groove;
wherein the plurality of first recesses (1200) aligns with the plurality of second recesses (1205) or the plurality of first recesses (1200) are offset from the plurality of second recesses (1205).

10. An apparatus (500) for supporting an elongated medical device (220), the apparatus (500) **characterized by**:
a first jaw (120) having a first surface (130);
a second jaw (122) having a second surface (131), the second surface (131) facing the first surface (130) of the first jaw (120);
a first pad (125B) arranged on the first surface (130) of the first jaw (120); and
a second pad (133B) arranged on the second surface (131) of the second jaw (122);
wherein
a first surface (901) of the first pad (125B) faces a second surface (902) of the second pad (133B),
the first pad (125B) includes a first wall (900) extending at least from a first outer edge (905) of the first surface (901) of the first pad (125B) toward the second surface (902) of the second pad (133B), and
the second pad (133B) includes a second wall (915) extending at least from a first outer edge (920) of the second surface (902) of the second pad (133B) toward the first surface (901) of the first pad (125B).

11. The apparatus (500) of claim 10, **characterized in that**:
the first pad (125C) includes a third wall (1000) extending at least from a second outer edge (910C) of the first surface (901C) of the first pad (125C) toward the second surface (902C) of the second pad (133C); and
the second pad (133C) includes a fourth wall (1005) extending at least from a second outer edge (925C) of the second surface (902C) of the second pad (133C) toward the first surface (901C) of the first pad (125C).

12. The apparatus (500) of claim 11, **characterized in that**:
the first wall (900C) extends along at least a portion of the first outer edge (905C) of the first surface (901C) of the first pad (125C);
the second wall (915C) extends along at least a portion of the first outer edge (920C) of the second surface (902C) of the second pad (133C);
the third wall (1000) extends along at least a portion of the second outer edge (910C) of the first surface (901C) of the first pad (125C); and
the fourth wall (1005) extends along at least a portion of the second outer edge (925C) of the second surface (902C) of the second pad (133C).

13. The apparatus (500) of claim 11, **characterized in that**:
the first wall (1100), the second wall (1105), the third wall (1115) and the fourth wall (1120) are castellated walls having a plurality of battlements (1110).

14. The apparatus (500) of any of claims 10-13, **characterized in that**:
the first pad (125B, 125C, 125D) and the second pad (133B, 133C, 133D) are configured to engage the elongated medical device (220), and to maintain the elongated medical device (220) at a center portion of the first pad (125B, 125C, 125D) and the second pad (133B, 133C, 133D) during movement of the elongated medical device (220).

15. An apparatus (500) for supporting an elongated medical device (220), the apparatus (500) **characterized by**:
a first jaw (120) having a first surface (130);
a first pad (125) arranged on the first surface (130) of the first jaw (120);
a second jaw (122) having a second surface (131), the second surface (131) being opposite the first surface (130); and
a second pad (133) arranged on the second surface (131) of the second jaw (122), the first pad (125) and the second pad (133) configured to maintain the elongated medical device (220) at a center portion of the first pad (125) and the second pad (133) when engaged with the elongated medical device (220).
